# EUROPÄISCHE PATENTSCHRIFT

(11) **EP 1 522 303 B1**
(45) Veröffentlichungstag und Bekanntmachung des Hinweises auf die Patenterteilung: **25.08.2010**
(21) Anmeldenummer: 04023545.9
(22) Anmeldetag: 02.10.2004
(51) Int. Cl.: A61K 8/37, A61Q 17/04

(54) **Verwendung von Dioldimerfettsäureestern**
Use of dioldimer fatty acid esters
Utilisation de diodimère d'esters d'acides gras

(30) Priorität: 10.10.2003 DE 10347218
(43) Veröffentlichungstag der Anmeldung: 13.04.2005
(73) Patentinhaber: Cognis IP Management GmbH, 40589 Düsseldorf (DE)
(72) Erfinder: Busch, Stefan, 46047 Oberhausen (DE); Kawa, Rolf, 40789 Monheim (DE); Westfechtel, Alfred, 40724 Hilden (DE)

(56) Entgegenhaltungen:
- DE-A- 10 162 697
- FR-A- 2 795 309

## Beschreibung

### Gebiet der Erfindung

Die Erfindung befindet sich auf dem Gebiet der Kosmetik und betrifft die Verwendung von Estern von Diolen und Dimerfettsäuren zur Herstellung von kosmetischen und pharmazeutischen Zubereitungen, speziell von Sonnenschutzmitteln mit verbesserter Wasserbeständigkeit.

### Stand der Technik

Obwohl aus dem Stand der Technik unzählige Emulsionen mit unterschiedlichsten Zusammensetzungen und Eigenschaften hinlänglich bekannt sind, bestehen im Bereich der Kosmetik nach wie vor intensive Bemühungen, sowohl die Stabilität als auch die sensorischen Eigenschaften dieser dispersen Systeme weiter zu verbessern. Zu den gegenwärtigen Trends gehört unter anderem die Suche nach neuen Ölkörpern und Polymeren, die sich leicht in Emulsionen einarbeiten lassen, die Formulierung besonders lagerstabiler Emulsionen erlauben und sensorisch ein leichteres Hautgefühl vermitteln.

Für spezielle Applikationsbereiche, wie beispielsweise Sonnenschutzmittel, spielt weiterhin die Wasserfestigkeit der Mittel eine wesentliche Rolle, da die Lichtschutzfilter möglichst lange auf der Haut verweilen sollten, ohne beim Baden abgewaschen zu werden. Die Wasserfestigkeit einer Sonnenschutzformulierung wird üblicherweise durch den Zusatz von Polymeren, wie beispielsweise PVP/Hexadecene Copolymer (Antaron® V-216), erzielt. Diese Polymere haben aber den Nachteil, dass die Wasserfestigkeit nur über einen kurzen Zeitraum sichergestellt wird, und ein Langzeitschutz, wie er z. B. von Wassersportlern (Surfern) oder für den Sonnenschutz für Kinder gefordert wird, nicht erreichbar ist. Darüber hinaus wird die Sensorik der Emulsion hinsichtlich Einziehvermögen, Verteilbarkeit und Klebrigkeit deutlich verschlechtert.

In diesem Zusammenhang sei auf die Französische Patentanmeldung FR 2795309 A1 (Nippon Fine Chemicals) hingewiesen, aus der kosmetische Zubereitungen bekannt sind, die als Ölkörper Dimerdioldimerfettsäureester enthalten. Bei den Mitteln handelt es sich überwiegend um dekorative Kosmetik, daneben werden aber auch Haar- und Hautbehandlungsmittel offenbart. Die Druckschrift besitzt jedoch keinen Bezug zum Thema Sonnenschutz und Wasserfestigkeit.

Die Aufgabe der vorliegenden Erfindung hat somit darin bestanden, Emulsionen auf neuartiger Polymer-Basis zur Verfügung zu stellen, die den Emulsionen eine verbesserte Sensorik, insbesondere hinsichtlich Einziehvermögen, Verteilbarkeit und Klebrigkeit verleihen. Ein weiterer Teilaspekt der Aufgabe bestand darin, Formulierungen zu entwickeln, die im Vergleich zum Stand der Technik eine verbesserte Wasserfestigkeit aufweisen, die also bei Einarbeitung von Lichtschutzfiltern einen verbesserten Langzeitschutz bieten.

### Beschreibung der Erfindung

Gegenstand der Erfindung sind ist die Verwendung von Dioldimerfettsäureestern im allgemeinen und speziell Dimerdioldimerfettsäureestern in kosmetischen oder pharmazeutischen Zubereitungen zur Verbesserung der Wasserfestigkeit.

Überraschenderweise wurde gefunden, dass kosmetische Zubereitungen auf Basis von Dioldimerfettsäureestern und insbesondere Dimerdioldimerfettsäureestern eine verbesserte Sensorik hinsichtlich Verteilbarkeit, Einziehvermögen und Klebrigkeit aufweisen. Die Ester lassen sich leicht in Emulsionen einarbeiten und führen insbesondere zu einer verbesserten Wasserfestigkeit der erfindungsgemäßen Mittel. Dabei wird vor allem auch der Marktstandard Antaron® V 216 deutlich überschritten.

### Dioldimerfettsäureester

Die in der Erfindung zu verwendenden Ester lassen sich nach den allgemeinen Prinzipien für die Veresterung von mehrwertigen Alkoholen mit mehrwertigen Carbonsäuren herstellen. Als Ausgangsstoffe werden Diole und Dimerfettsäuren eingesetzt, die üblicherweise unter Rückfluss und gleichzeitigem Entfernen des Reaktionswassers miteinander umgesetzt werden. Ester- und Säurekomponente stellen beide industriell verfügbare Handelsprodukte dar, die beispielsweise von der Cognis Deutschland GmbH & Co. KG erhältlich sind. Die Dimerfettsäuren werden dabei nach bekannten Verfahren der präparativen organischen Chemie durch Kondensation ungesättigter linearer Fettsäuren mit 16 bis 22 Kohlenstoffatomen, speziell der Ölsäure erhalten; herstellungsbedingt können sich auch geringe Mengen höherer Kondensationsprodukte, insbesondere Trimere enthalten. Als Alkoholkomponenten kommen einerseits aliphatische, lineare oder verzweigte Diole mit 2 bis 36 Kohlenstoffatomen in Betracht, wobei die Moleküle vorzugsweise über zwei primäre Hydroxylgruppen verfügen.

Typische Beispiele sind 1,6-Hexandiol, 1,8-Octandiol, 1,10-Decandiol, 1,12-Dodecandiol, 1,14-Tetradecandiol, 1,16-Hexadecandiol, 1,18-Octadecandiol sowie insbesondere die verzweigten Dimerdiole, welche durch Reduktion der Säurefunktionen der entsprechenden Dimerfettsäuren hergestellt werden. Alternativ kann sich die Alkoholkomponente auch von Alkylenglycolen ableiten, die 1 bis 20 und vorzugsweise 1 bis 5 Alkylenoxideinheiten, speziell Ethylen- und/oder Propylenoxideinheiten aufweisen. Typische Beispiele sind Ethylenglycol, Ethylendiglycol, Propylenglycol, Propylendiglycol sowie deren Gemische.

Besonders bevorzugt sind Ester, die sich von Diolen, speziell Dimerdiolen und Dimerfettsäuren ableiten, welche letztlich durch Kondensation von Ölsäure erhalten worden sind und daher sowohl im Alkohol- als auch im Säureteil 36 Kohlenstoffatome aufweisen. Bei der Kondensation von Disäure und Diol reagiert die Dimerfettsäure nicht ausschließlich selektiv mit den Hydroxylgruppen von zwei Diolmolekülen ab, es kommt parallel natürlich auch zur Reaktion, bei der die beiden Hydroxylgruppen des Diols mit den Säuregruppen zweier Dimerfettsäuremoleküle zusammentreten. Dem molaren Einsatzverhältnis der Einsatzstoffe und damit der Statistik folgend, werden daher sowohl Mono- und Diester als auch Oligomere und Polymere gebildet. Das Reaktionsprodukt stellt somit stets eine Mischung dar, die im wesentlichen über die Hydroxyl- und die Säurezahl gekennzeichnet werden kann. Vorzugsweise werden des weiteren solche Ester eingesetzt, die
- ein mittleres Molekulargewicht im Bereich von 1.000 bis 50.000, insbesondere 5.000 bis 25.000 Dalton,
- eine Hydroxylzahl im Bereich von 50 bis 150, vorzugsweise 60 bis 120 und/oder
- eine Säurezahl im Bereich von 0,1 bis 5, vorzugsweise 0,5 bis 1,5
aufweisen.

### Sonnenschutzmittel

Typische Sonnenschutzmittel stellen Emulsionen dar, bei denen es sich sowohl um Öl-in-Wasser- als auch Wasser-in-Öl-Typen handeln kann; auch multiple W/O/W- oder O/W/O-Emulsionen sind möglich. Gemein ist diesen Mitteln, dass sie als weiteren Bestandteil wenigstens einen UV-Lichtschutzfaktor und/oder ein Feuchthaltemittel enthalten.

### UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
- Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan® AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden) sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000, Eusolex® T, Eusolex® T-ECO, Eusolex® T-S, Eusolex® T-Aqua, Eusolex® T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE® oder Z-COTE HP1® verwendet.

### Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Bevorzugt geeignet als Feuchthaltemittel sind Glycerin, Diglycerin, Triglycerin und Butylenglycol.

### Gewerbliche Anwendbarkeit

Wie schon erläutert, besteht ein wesentliches Merkmal der Erfindung darin, dass der Zusatz von Dioldimerfettsäureestern und insbesondere Dimerdioldimerfettsäureestern die Eigenschaften kosmetischer Produkte nachhaltig steigert. Weitere Gegenstände der Erfindung betreffen daher die Verwendung dieser Ester zur Verbesserung der Wasserfestigkeit und sensorischen Eigenschaften von kosmetischen und/oder pharmazeutischen Zubereitungen im allgemeinen sowie die Herstellung von Sonnenschutzmitteln im besonderen. Die genannten Effekte eignen sich dabei insbesondere auch für die Formulierung von Insektenschutzmitteln und Deodorants bzw. Antitranspirantien.

Die Sonnenschutzmittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia@ SP von Cognis;
- Polyalkylenglycole sowie
- Glycerincarbonat.

### • Alkoxylate

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### • Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### • Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### • Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### • Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care@ 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### • Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### • Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylatund eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-snglycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminiumund/oder Zinkstearat bzw. ―ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar@ C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### • Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### • Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder ―phosphate, wie beispielsweise Lanosterin―, Cholesterin―, Campesterin―, Stigmasterin― und Sitosterinsulfat bzw ―phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### • Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

### • Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl)piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Insekten-Repellentien, Selbstbräuner und Depigmentierungsmittel

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

### Beispiel 1

In einer 2-1-Rührapparatur mit Kühler wurden 458 g (0,8 Mol) Dimerfettsäure (Pripol® 1009) und 770 g (1,4 Mol) Dimerdiol (Pripol® 2033) vorgelegt und bei 240 °C unter Stickstoffabdeckung unter Rückfluss erhitzt und dabei das Kondensationswasser kontinuierlich abgetrennt. Nach Abschluss der Reaktion wurde bei gleichbleibender Temperatur der Druck in der Apparatur auf 10 mbar abgesenkt und 1 h weiter gerührt. Nach dem Brechen des Vakuums ließ man den Ansatz abkühlen und erhielt 1198 g (entsprechend 99 % der Theorie) Dimerdioldimerfettsäureester mit niedriger Hydroxylzahl in Form einer farblosen Flüssigkeit, die die folgenden Kennzahlen aufwies : Säurezahl (nach ISO 660) : 1,4; Verseifungszahl (nach ISO 3657) : 76 und Hydroxylzahl (nach ISO 4326) : 60.

### Beispiel 2

Analog Beispiel 1 wurden 286,3 g (0,5 Mol) Dimerfettsäure und 825,2 g (1,5 Mol) Dimerdiol zur Reaktion gebracht. Man erhielt 1092 g (entsprechend 99 % der Theorie) Dimerdioldimerfettsäureester mit hoher Hydroxylzahl in Form einer farblosen Flüssigkeit, die die folgenden Kennzahlen aufwies : Säurezahl (nach ISO 660) : 1,4; Verseifungszahl (nach ISO 3657) : 52 und Hydroxylzahl (nach ISO 4326) : 105.

### Bestimmung der Wasserfestigkeit der Dimerdioldimerfettsäureester

Die Dimerdioldimerfettsäureester wurden in Basisrezepturen eingearbeitet und die Wasserfestigkeit der erfindungsgemäßen Mittel bestimmt. Zur Bestimmung der Wasserfestigkeit wurde eine definierte Menge der Zubereitungen (gemäß Tabelle 1) auf ein geeignetes Trägermaterial aufgetragen und in einem Becherglas nach vorgegebenen Kriterien gewässert, wobei mittels eines Magnetrührers das Wasser in Bewegung gehalten wurde. Der SPF *(Sun protection factor)* wurde vor und nach dem Wässern mit dem UV-1000S Labsphere Ultraviolet Transmittance Analyser bestimmt. Die sensorische Beurteilung erfolgte durch ein Panel zehn geschulter Probanden, die Noten von (1) = sehr gut bis (6) = ungenügend vergaben. Angegeben sind Mittelwerte aus jeweils drei Messungen.

### Wasserfestigkeit:

- Trägermaterial: Vitro-Skin N19, Firma IMS (4 x 3 cm) auf Diarahmen
- Auftragsmenge: 2 mg/cm²
- Trockenzeit vor der 1. Messung: 15 min, Temperatur 30 °C
- Wassertemperatur: 23 °C (16 °d)
- pH-Wert Wasser: 7,0 +/- 0,5
- Wassermenge: 400 ml
- Rührgeschwindigkeit: 300 UpM (Magnetrührer)
- Wässerungszeit: 2 x 20 min mit einer Pause von 20 min
- Trockenzeit vor der 2. Messung: 15 min, Temperatur 30 °C

Die Ergebnisse sind in Tabelle 1 zusammengefasst. Die Beispiele 1 und 2 sind erfindungsgemäß, die Beispiele V1 und V2 dienen zum Vergleich. Die Mengenangaben in nachfolgenden Beispielen beziehen sich, soweit nicht anders angegeben, auf Gew.-% der handelsüblichen Substanzen in der Gesamtzusammensetzung.

**Tabelle 1:**

| **Basisrezepturen von Sonnenschutzformulierungen; Wasserfestigkeit und Sensorik** | | | | |
|---|---|---|---|---|
| **Zusammensetzung / Performance** | **1** | **2** | **V1** | **V2** |
| Eumulgin® VL 75 | 4,0 | 4,0 | 4,0 | 4,0 |
| Myritol® 331 | 7,0 | 7,0 | 7,0 | 7,0 |
| Cetiol® OE | 6,0 | 6,0 | 6,0 | 6,0 |
| Eutanol® G 16 | 3,0 | 3,0 | 3,0 | 3,0 |
| Dimerdioldimerfettsäureester gem. Bsp. 1 | 4,0 | 2,0 | - | - |
| Antaron® V 220 | - | 2.0 | 4,0 | - |
| Antaron® V 216 | - | - | - | 4.0 |
| Neo Heliopan® AV | 7,5 | 7,5 | 7,5 | 7,5 |
| Parsol® 1789 | 2,0 | 2,0 | 2,0 | 2,0 |
| Carbopol® 2984 | 0,2 | 0,2 | 0,2 | 0,2 |
| Glycerin | 5,0 | 5,0 | 5,0 | 5,0 |
| Wasser/NaOH/Konservierungsmittel | ad 100/pH = 7/ q.s | | | |

| ***In-vitro Sun Protection Factor (SPF)*** | | | | |
|---|---|---|---|---|
| - vor der Wasserbehandlung | 15 | 15 | 15 | 15 |
| - nach der Wasserbehandlung | 15 | 14 | 9 | 10 |
| - Differenz (%-rel.) | 100 | 93 | 60 | 66 |

| ***Sensorische Beurteilung*** | | | | |
|---|---|---|---|---|
| Einziehvermögen | 1 | 1 | 5 | 4 |
| Glätte | 1 | 1 | 4 | 4 |
| Klebrigkeit | 1 | 2 | 6 | 5 |

Die Beispiele 1 und 2 zeigen, dass die Dimerdioldimerfettsäureester im Vergleich zu den Standardprodukten Antaron® V 220 und V 216 den Sonnenschutzformulierungen eine deutlich höhere Wasserfestigkeit verleihen. Gleichzeitig zeichnen sich Rezepturen durch eine deutlich bessere sensorische Beurteilung aus.

In den nachfolgenden Tabelle werden eine Reihe von Formulierungsbeispielen gegeben. Mengenangaben beziehen sich auf Gew.-% der handelsüblichen Substanzen in der Gesamtzusammensetzung. Die Abkürzung "L" steht dabei für Lotion, "C" für Creme und "S" für Spray.

**Tabelle 2**

| **O/W-Sonnenschutzemulsionen** | | | | | | | | | | | |
|---|---|---|---|---|---|---|---|---|---|---|---|
| **Komponente** | **1** | **2** | **3** | **4** | **5** | **6** | **7** | **8** | **9** | **10** | **11** |
| | S | C | L | C | C | C | C | L | L | C | L |
| Eumulgin® VL 75 | | | | | | 4 | 4 | 2 | | | |
| Eumulgin® B2 | 2 | | | | | | | 2 | | | |
| Tween® 60 | | | | 1 | | | | | | | |
| Myrj® 51 | | 3 | | 2 | | | | | | | |
| Cutina® E 24 | 1 | | | 1 | | | | | | | |
| Hostaphat® KL 340 N | | | | | | | | | 2 | | |
| Lanette® E | | | 0.5 | | | | | 0.5 | | | |
| Amphisol® K | | | 1 | | | 1 | | 0.5 | | 1 | |
| Natriumstearat | | | | | | | 1 | | | | 2 |
| Emulgade® PL 68/50 | | | 1 | | 5 | | | | | 4 | |
| Tego® Care 450 | | | | | | | | | | 3 | |
| Cutina® MD | 2 | | | 6 | | | 4 | | | 6 | |
| Lanette® 14 | 1 | | | 1 | | | | 2 | | | 4 |
| Lanette®O | 1 | 6 | | | 5 | 2 | | 2 | | | |
| Dimerdiolfettsäureester gem. Bsp. 1 | 2 | 2 | 4 | 1 | 2 | 2 | 2 | 1 | 2 | 2 | 1 |
| Emery® 1780 | | | | | 0.5 | 0.5 | | | | | |
| Lanolin, wasserfrei USP | | | | | | | 5 | | | | |
| Myritol® PC | | | | | 5 | | | | | | |
| Myritol® 331 | 5 | | 8 | | | 6 | | 10 | | 2 | |
| Finsolv® TN | | | 1 | | | | | 1 | 8 | | |
| Cetiol® CC | | 2 | 5 | | | 4 | 4 | 2 | | 2 | |
| Cetiol® OE | | | 3 | | | | | | 2 | 3 | |
| Dow Corning DC® 244 | 4 | | 1 | | 5 | | | 2 | | | 2 |
| Dow Corning DC® 2502 | | 1 | | | 2 | | | | | | |
| Squatol® S | | | | | | | 4 | | | | |
| Silikonöl Wacker AK® 350 | | 2 | | | | | | | | | |
| Cetiol® 868 | | | | | 2 | | 4 | | | | 7 |
| Cetiol® J 600 | | | | | 3 | 2 | | | | 5 | |
| Mineralöl | | | | 9 | | | | | | | |
| Cetiol® B | | | 1 | | | | | | | 2 | |
| Eutanol® G | | | | | | | | | | | |
| Eutanol® G 16 | | | | | | | | | | | |
| Cetiol® PGL | | 5 | | | | | | | | 5 | |
| Mandelöl | | | 2 | | | | 1 | | | | |
| Photonyl® LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Neo Heliopan® Hydro (Na-Salz) | 2 | | 2.2 | | 3 | 3 | | | | | 2 |
| Neo Heliopan® AP (Na-Salz) | 0.5 | | | | | | | | | | 1 |
| Neo Heliopan® 303 | 3 | 5 | 9 | 4 | | | | | | | |
| Neo Heliopan® BB | | | | | 1 | | | | | | 2 |
| Neo Heliopan® MBC | 2 | | | 3 | | 2 | 2 | 2 | | | 1 |
| Neo Heliopan® OS | | | | | | | | | 10 | 7 | |
| Neo Heliopan® E 1000 | | 7.5 | | 6 | | | | | | | 6 |
| Neo Heliopan® AV | | | 7.5 | | | 7.5 | 4 | 5 | | | |
| Uvinul® T 150 | 2 | | | | 2.5 | | | 1 | | | |
| Uvinul® A PLUS | | | | 1 | | | | 1 | | | |
| Parsol® 1789 | 1 | 1 | 1 | | | | 2 | | 2 | 2 | |
| Zinkoxid NDM | | | 5 | | | 10 | | 3 | | 5 | 4 |
| Eusolex® T 2000 | | | | | 5 | | 3 | 3 | | | 4 |
| Veegum® Ultra | | | 0.75 | | | | | 1 | 1 | | |
| Keltrol® T | | | 0.25 | | | | | 0.5 | 0.5 | | |
| Carbopol® 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 3 | 3 | | 2 | 5 | 2 | | 2 |
| Glycerin | 5 | 5 | 5 | | 3 | 3 | 2 | | 4 | | 3 |
| Konservierungsmittel, NaOH Wasser | q.s. ad 100 | | | | | | | | | | |

**Tabelle 3**

| **O/W-Sonnenschutzemulsionen** | | | | | | | | | | | |
|---|---|---|---|---|---|---|---|---|---|---|---|
| **Komponente** | **12** | **13** | **14** | **15** | **16** | **17** | **18** | **19** | **20** | **21** | **22** |
| | L | C | L | C | C | C | L | C | C | L | S |
| Eumulgin® VL 75 | 4 | 3 | 4.5 | | 3 | | | | 4 | | |
| Eumulgin® B2 | | | | | | | | | | 1 | |
| Tween® 60 | | | | | | | | | | 1 | |
| Cutina® E 24 | | | | 2 | | | | | | | |
| Hostaphat® KL 340 N | | | | | | | | | | 0.5 | |
| Lanette® E | 0.5 | | 0.5 | 0.5 | | | 0.1 | | 0.5 | | |
| Amphisol® K | 0.5 | | | | | 1 | 1 | 1 | | | |
| Natriumstearat | | | | | 1 | | | | | | |
| Emulgade® PL 68/50 | | 6 | | | | 4.5 | 1 | 5 | | | |
| Tego® Care 450 | 1 | | | | | | | | 4 | | |
| Cutina® MD | 1 | | | 8 | 6 | 1 | | | | 4 | 1 |
| Lanette® 14 | | 2 | | | | | | 2 | | 1 | |
| Lanette® O | | | | 2 | | | | | 1 | 1 | |
| Dimerdiolfettsäureester gem. Bsp. 1 | 4 | 2 | 4 | 1 | 1 | 4 | 2 | 2 | 2 | 1 | 3 |
| Myritol® PC | | | | | | | | | 5 | | |
| Myritol® 331 | 12 | | 12 | | | 8 | 8 | | | 10 | 8 |
| Finsolv® TN | | | | | 5 | | | 3 | 3 | | |
| Cetiol® CC | 6 | | 6 | | | 5 | 5 | | | | |
| Cetiol® OE | | | | | 2 | | | | | | 2 |
| Dow Corning DC® 244 | | 2 | | | 1 | | | | | | |
| Dow Corning DC® 2502 | | 1 | | | 1 | | | | | | |
| Ceraphyl® 45 | | | | | | | | | | 2 | 2 |
| Silikonöl Wacker AK® 350 | | | | | 1 | | | | | | |
| Cetiol® 868 | | 2 | | | | | | | | | |
| Cetiol® J 600 | | 2 | | | | | | | | | |
| Mineralöl | | | | 10 | | | | | | | |
| Cetiol® B | 4 | | 4 | | | | | 4 | | | |
| Eutanol® G | | 3 | | | | 3 | | | | | |
| Eutanol® G 16 S | 10 | | | | | | | | | | |
| Cetiol® PGL | | | | | | | | | 2 | | |
| Photonyl® LS | | | | | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Insect Repellent 3535 | | | | 7 | | | | | | | |
| Neo Heliopan® Hydro (Na-Salz) | | | | | | | | | | 3 | |
| Eusolex® OCR | 6 | | 9 | | 5 | 7 | 9 | | 4 | | 7 |
| Neo Heliopan® BB | | | | | | | | 1 | 1 | | 1 |
| Neo Heliopan® MBC | | 2 | | 1 | | | | 3 | 1 | | 3 |
| Neo Heliopan® OS | 2 | | | | | | | | 7 | | |
| Neo Heliopan® E1000 | | 4 | | | | | | 5 | | | |
| Neo Heliopan® AV | | 4 | 7.5 | 5 | | | | 5 | 4 | 7.5 | |
| Uvinul® T 150 | 1 | | | | | | | | 1.3 | 1 | 1 |
| Parsol® 1789 | 1 | | | | | | | | 2 | | 1 |
| Z-Cote® HP 1 | 7 | 2 | 5 | | | 7 | 5 | | 6 | 2 | |
| Eusolex® T 2000 | 5 | 2 | | | 10 | | | 10 | | 2 | |
| Veegum® Ultra | 1.5 | | 1.5 | | | 1.5 | 1.2 | | 1 | | |
| Keltrol® T | 0.5 | | 0.5 | | | 0.5 | 0.4 | | 0.5 | | |
| Cosmedia® SP | | 0.3 | | | | | 0.1 | | | | 0.3 |
| Pemulen® TR 2 | | | | 0.3 | | | | 0.2 | | | |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Konservierungsmittel, NaOH Wasser | q.s. ad 100 | | | | | | | | | | |

**Tabelle 4**

| **W/O-Sonnenschutzemulsionen** | | | | | | | | | | | |
|---|---|---|---|---|---|---|---|---|---|---|---|
| **Komponente** | **23** | **24** | **25** | **26** | **27** | **28** | **29** | **30** | **31** | **32** | **33** |
| | C | L | C | L | L | C | C | C | L | C | C |
| Dehymuls® PGPH | 4 | 2 | 1 | 3 | 3 | 1 | 1 | 2 | 2 | 4 | 1 |
| Monomuls® 90-O18 | | | 2 | | | | | | | | |
| Lameform® TGI | 2 | | 4 | | 3 | | | | | 1 | 3 |
| Abil® EM 90 | | | | | | | 4 | | | | |
| Glucate® DO | | | | | | | | | | | 3 |
| Isolan® PDI | | | | | | 4 | | 2 | | | |
| Arlacel® 83 | | | | 2 | | | | | | | |
| Elfacos® ST9 | | | | | | | | | 2 | | |
| Elfacos® ST37 | | | | | | | | | | | |
| Arlacel® P 135 | | 2 | | | | | | | | | |
| Dehymuls® HRE 7 | | | | | | | | | | | |
| Zinkstearat | 1 | | | 1 | 1 | | | 1 | | 1 | |
| Microkristallines Wachs | | | 5 | 2 | | | | | | | 5 |
| Bienenwachs | 1 | | | 1 | | | | 5 | | 7 | |
| Tego® Care CG | | | | | 1 | | | | | | .5 |
| Prisorine® 3505 | 1 | | 1 | 1 | | 1 | 1 | | | | 1 |
| Dimerdiolfettsäureester gem. Bsp.1 | 3 | 4 | 2 | 1 | 1 | 2 | 2 | 2 | 3 | 1 | 1 |
| Emery® 1780 | | | 5 | | | | | | | 4 | |
| Wollwachsalkohol, wasserfrei, USP | | | | | | | | | | | 1 |
| Myritol® PC | | | | | 3 | | | 4 | | | |
| Myritol® 331 | 10 | | | | 3 | 6 | | | | | 8 |
| Finsolv® TN | | | | 5 | | | 5 | | | | |
| Cetiol® CC | 12 | 12 | | | | 2 | | | 2 | | 5 |
| Cetiol® OE | | | | | 4 | | 5 | | 4 | 2 | |
| Dow Corning DC® 244 | | | | | | | 2 | | | | |
| Dow Corning DC® 2502 | | | 1 | | 2 | | | | | | |
| Prisorine® 3758 | | | | | | | | | | 2 | |
| Silikonöl Wacker AK® 350 | | | | 4 | | | | 3 | | | |
| Cetiol® 868 | | | | | | | | | | 2 | |
| Eutanol® G 16 | | 3 | | | | | | | | | |
| Eutanol® G 16S | | | | | | | | | | | |
| Cetiol® J 600 | | | 4 | 2 | | | | | | | |
| Ceraphyl® 45 | | | | 2 | | | | 2 | | 6 | |
| Mineralöl | | | | | 4 | | | | | | |
| Cetiol® B | | | 2 | 4 | | | | | | 3 | |
| Eutanol® G | | | 3 | | | | | 8 | | | |
| Cetiol® PGL | | 11 | | | | 4 | | | 9 | | |
| Mandelöl | | | | | 1 | | 5 | | | | |
| Photonyl® LS | | 2 | | 1 | | | | | 4 | | |
| Panthenol | 1,0 | | | | | | | | | | |
| Bisabolol | 0,2 | | | | | | | | | | |
| Tocopherol / Tocopherylacetat | 1,0 | | | | | | | | | | |
| Magnesiumsulfat x 7 Wasser | 1 | | | | | | | | | | |
| Neo Heliopan^{®} Hydro (Na-Salz) | | 2 | | 3 | | | | | | | |
| Neo Heliopan^{®} AP (Na-Salz) | 0.5 | | | | | | | | | | 1 |
| Neo Heliopan^{®} 303 | | | | | 4 | | | | | 6 | |
| Neo Heliopan^{®} BB | | 4 | 2 | | | | 2 | | | | |
| Neo Heliopan^{®} MBC | | | | | | | | 4 | | 3 | |
| Neo Heliopan^{®} OS | | | | | | | | | | | |
| Neo Heliopan^{®} E 1000 | | | | | | | | | 5 | | |
| Neo Heliopan^{®} AV | | 3 | 6 | 6 | | 7.5 | 7.5 | | 5 | | 7.5 |
| Uvinul^{®} A PLUS | | | | 1 | | | | 1 | | | |
| Uvinul^{®} T 150 | | | | | 2.5 | | | 1 | | 2 | |
| Parsol^{®} 1789 | | 2 | | | | | | 1 | | 2 | |
| Zinkoxid NDM | | | | | | 6 | | | | | |
| Eusolex^{®} T 2000 | 15 | | 10 | | 5 | | 4 | | | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Konservierungsmittel, NaOH Wasser | q.s. ad 100 | | | | | | | | | | |

**Tabelle 5**

| **W/O-Sonnenschutzemulsionen** | | | | | | | | | | | |
|---|---|---|---|---|---|---|---|---|---|---|---|
| **Komponente** | **34** | **35** | **36** | **37** | **38** | **39** | **40** | **41** | **42** | **43** | **44** |
| | L | C | L | L | L | L | L | L | L | C | L |
| Dehymuls® PGPH | 3 | 1 | 5 | 1 | 3 | 3 | 2 | 4 | 0.5 | 1 | 4 |
| Monomuls® 90-018 | | 1 | | | | | | | | | |
| Lameform® TGI | | | | | 1 | | | 1 | | 3 | 1 |
| Abil® EM 90 | | | | 1 | | | | | | 2 | |
| Glucate® DO | | | | 3 | | 2 | | | | | |
| Isolan® PDI | | 3 | | | | | 4 | | | | |
| Arlacel® 83 | | | | | | 3 | | | | | |
| Elfacos® ST9 | | | | | | | | | | | 2 |
| Elfacos® ST37 | 2 | | | | | | | | | | |
| Arlacel® P 135 | | | | | | 3 | | | | | |
| Dehymuls® HRE 7 | | | | | | | | | 4 | | |
| Zinkstearat | | 2 | 2 | 1 | 1 | | | 1 | 1 | | |
| Mikrokristallines Wachs | | | | | 4 | | 1 | | | 4 | |
| Bienenwachs | | 4 | | 2 | | | 1 | | 2 | | 1 |
| Tego® Care CG | | | | | | | | | | | |
| Isostearinsäure | 1 | 1 | | | | | 1 | 1 | | 1 | 1 |
| Dimerdiolfettsäureester gem. Bsp. 1 | 2 | 4 | 3 | 3 | 2 | 2 | 1 | 3 | 3 | 1 | 4 |
| Emery® 1780 | | 7 | 3 | | | | | | | | |
| Wollwachsalkohol, wasserfrei, USP | | | | | | | | | | | |
| Myritol® PC | | | | | | | | | | | |
| Myritol® 331 | 4 | 2 | 3 | | 5 | | | 8 | 5 | 4 | |
| Finsolv® TN | | 5 | 5 | | | 7 | | | | | |
| Cetiol® CC | 3 | 1 | | | | | 3 | 16 | | | 12 |
| Cetiol® OE | | 3 | | 2 | | | 3 | | | | |
| Dow Corning DC® 244 | | 4 | | 2 | | | | | | | |
| Dow Corning DC® 2502 | | | | 1 | | | | | | | |
| Prisorine® 3578 | | 1 | | | | | | | | | |
| Silikonöl Wacker AK® 350 | | | | 1 | | | | | | | |
| Cetiol® 868 | | | | | | | | | | | |
| Eutanol® G 16 | | | | | | | | | | | 3 |
| Eutanol® G 16S | | | | | | | | | | | 7 |
| Cetiol® J 600 | | | | 3 | | | | | | | |
| Ceraphyl® 45 | | | | 1 | | | | | 5 | 4 | |
| Mineralöl | | | | | | | 9 | | | | |
| Cetiol® B | | | | | 3 | 3 | | 2 | 2 | | |
| Eutanol® G | | | | 2 | | | | | 5 | | |
| Cetiol® PGL | | | | | | | | 2 | | | |
| Mandelöl | | | 2 | | | | | | | | |
| Insect Repellent 3535 | | | | | 7 | | | | | | |
| Photonyl® LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan® Hydro (Na-Salz) | | 4 | | | | | | 4 | | | |
| Neo Heliopan® 303 | 6 | 2 | | | | | | | 6 | | |
| Neo Heliopan® BB | | 2 | | 2 | | 2 | | | | | |
| Neo Heliopan® MBC | 2 | | | | 3 | | 4 | | 2 | | |
| Neo Heliopan® OS | | | | | 10 | 8 | | | | | |
| Neo Heliopan® E 1000 | | | 5 | 6 | | | | | | 5 | |
| Neo Heliopan® AV | | 5 | 5 | | | 7.5 | | | | 5 | |
| Uvinul® T 150 | 1 | | | 2 | 2 | | | | 3 | 2 | |
| Parsol® 1789 | | 1 | 1 | | | | 1 | | 0.5 | | |
| Z-Cote® HP 1 | 4 | 10 | | | | | | 5 | 5 | | |
| Titandioxid T 805 | | | | 2 | | 3 | | 7 | | 4 | 7 |
| Ethanol | | | | 8 | | 10 | | | | | |
| Butylenglykol | 5 | 1 | | 3 | 2 | | | | 8 | 2 | |
| Glycerin | | | 6 | 2 | | | 5 | 5 | | 3 | 5 |
| Konservierungsmittel, NaOH Wasser | q.s. ad 100 | | | | | | | | | | |

## Patentansprüche

1. Verwendung von Dioldimerfettsäureestern zur Verbesserung der Wasserfestigkeit von kosmetischen und/oder pharmazeutischen Zubereitungen.

2. Verwendung nach Anspruch 1, **dadurch gekennzeichnet, dass** sie Dioldimerfettsäureester verwendet werden, deren Alkoholkomponente sich von aliphatischen, linearen oder verzweigten Diolen mit 2 bis 36 Kohlenstoffatomen ableitet.

3. Verwendung nach Anspruch 2, **dadurch gekennzeichnet, dass** sich die Alkoholkomponente vom 1,6-Hexandiol, 1,8-Octandiol, 1,10-Decandiol, 1,12-Dodecandiol, 1,14-Tetradecandiol, 1,16-Hexadecandiol, 1,18-Octadecandiol oder Dimerdiolen ableitet.

4. Verwendung nach Anspruch 1, **dadurch gekennzeichnet, dass** sich die Alkoholkomponente von Polyalkylenglycolen mit 1 bis 20 Alkylenoxideinheiten ableitet.

5. Verwendung nach Anspruch 4, **dadurch gekennzeichnet, dass** sich die Alkoholkomponente von Ethylenglycol, Ethylendiglycol, Propylenglycol oder Propylendiglycol ableitet.

6. Verwendung nach mindestens einem der Ansprüche 1 bis 5, **dadurch gekennzeichnet, dass** die Dioldimerfettsäureester, ein mittleres Molekulargewicht im Bereich von 1.000 bis 50.000 Dalton aufweisen.

7. Verwendung nach mindestens einem der Ansprüche 1 bis 6, **dadurch gekennzeichnet, dass** sie die Dioldimerfettsäureester eine Hydroxylzahl im Bereich von 50 bis 150 aufweisen.

8. Verwendung nach mindestens einem der Ansprüche 1 bis 7, **dadurch gekennzeichnet, dass** die Dioldimerfettsäureester eine Säurezahl im Bereich von 0,1 bis 5 aufweisen.

9. Verwendung nach mindestens einem der Ansprüche 1 bis 8, **dadurch gekennzeichnet, dass** Dioldimerfettsäureester in Mengen von 0,1 bis 20 Gew.-% - bezogen auf die Endzubereitungen - verwendet werden.

10. Verwendung nach mindestens einem der Ansprüche 1 bis 9, **dadurch gekennzeichnet, dass** die Dioldimerfettsäureester zusammen mit mindestens einem UV-Lichtschutzfaktor und/oder einem Feuchthaltemittel verwendet werden.

## Claims

1. Use of diol dimer fatty acid esters for improving the water resistance of cosmetic and/or pharmaceutical preparations.

2. Use according to claim 1, **characterized in that** diol dimer fatty acid esters are used of which the alcohol component is derived from aliphatic, linear or branched C₂₋₃₆ diols.

3. Use according to claim 2, **characterized in that** the alcohol component is derived from hexane-1,6-diol, octane-1,8-diol, decane-1,10-diol, dodecane-1,12-diol, tetradecane-1,14-diol, hexadecane-1,16-diol, octadecane-1,18-diol or dimer diols.

4. Use according to claim 1, **characterized in that** the alcohol component is derived from polyalkylene glycols containing 1 to 20 alkylene oxide units.

5. Use according to claim 4, **characterized in that** the alcohol component is derived from ethylene glycol, ethylene diglycol, propylene glycol or propylene diglycol.

6. Use according to at least one of claims 1 to 5, **characterized in that** the diol dimer fatty acid esters have an average molecular weight of 1,000 to 50,000 Dalton.

7. Use according to at least one of claims 1 to 6, **characterized in that** the diol dimer fatty acid esters have a hydroxyl value of 50 to 150.

8. Use according to at least one of claims 1 to 7, **characterized in that** the diol dimer fatty acid esters have an acid value of 0.1 to 5.

9. Use according to least one of claims 1 to 8, **characterized in that** the diol dimer fatty acid esters are used in quantities of 0.1 to 20% by weight, based on the final preparations.

10. Use according to at least one of claims 1 to 9, **characterized in that** the diol dimer fatty acid esters are used together with at least one UV protection factor and/or one moisturizer.

## Revendications

1. Utilisation d'esters d'acides gras dimères et de diols pour améliorer la résistance à l'eau de compositions cosmétiques et/ou pharmaceutiques.

2. Utilisation selon la revendication 1, **caractérisée en ce qu'**on utilise des esters d'acides gras dimères et de diols dont le composant alcool est dérivé de diols aliphatiques, linéaires ou ramifiés comprenant 2 à 36 atomes de carbone.

3. Utilisation selon la revendication 2, **caractérisée en ce que** le composant alcool est dérivé de 1,6-hexanediol, de 1,8-octanediol, de 1,10-décanediol, de 1,12-dodécanediol, de 1,14-tétradécanediol, de 1,16-hexadécanediol, de 1,18-octadécanediol ou de diols dimères.

4. Utilisation selon la revendication 1, **caractérisée en ce que** le composant alcool est dérivé de polyalkylèneglycols comprenant 1 à 20 unités d'oxyde d'alkylène.

5. Utilisation selon la revendication 4, **caractérisée en ce que** le composant alcool est dérivé d'éthylèneglycol, d'éthylènediglycol, de propylèneglycol ou de propylènediglycol.

6. Utilisation selon au moins l'une quelconque des revendications 1 à 5, **caractérisée en ce que** les esters d'acides gras dimères et de diols présentent un poids moléculaire moyen dans la plage de 1000 à 50 000 Daltons.

7. Utilisation selon au moins l'une quelconque des revendications 1 à 6, **caractérisée en ce que** les esters d'acides gras dimères et de diols présentent un indice d'hydroxyle dans la plage de 50 à 150.

8. Utilisation selon au moins l'une quelconque des revendications 1 à 7, **caractérisée en ce que** les esters d'acides gras dimères et de diols présentent un indice d'acide dans la plage de 0,1 à 5.

9. Utilisation selon au moins l'une quelconque des revendications 1 à 8, **caractérisée en ce que** les esters d'acides gras dimères et de diols sont utilisés en des quantités de 0,1 à 20% en poids - par rapport aux compositions finales.

10. Utilisation selon au moins l'une quelconque des revendications 1 à 9, **caractérisée en ce que** les esters d'acides gras et de diols sont utilisés ensemble avec au moins un facteur de protection contre la lumière UV et/ou un agent de rétention de l'humidité.
